# EUROPEAN PATENT SPECIFICATION

(11) **EP 3 585 262 B1**
(45) Date of publication and mention of the grant of the patent: **28.06.2023**
(21) Application number: 18757133.6
(22) Date of filing: 26.02.2018
(51) Int. Cl.: A61B 5/282

(54) **EMERGENCY CARDIAC AND ELECTROCARDIOGRAM ELECTRODE PLACEMENT SYSTEM**
SYSTEM ZUR PLATZIERUNG VON NOTFALL-HERZ- UND ELEKTROKARDIOGRAMMELEKTRODEN
SYSTÈME DE PLACEMENT D'ÉLECTRODE CARDIAQUE ET D'ÉLECTROCARDIOGRAMME D'URGENCE

(30) Priority: 25.02.2017 US 201715904411; 01.03.2017 US 201762465752 P; 08.07.2017 US 201762530144 P; 22.12.2017 US 201715853578
(43) Date of publication of application: 01.01.2020
(73) Proprietor: CB Innovations, LLC, Escondido, CA 92029 (US)
(72) Inventor: DUNPHY, Stephen, Escondido, CA 92029 (US); McCLUNG, Christain, Escondido, CA 92029 (US); RONAN, Sean, Escondido, CA 92029 (US)
(74) Representative: Tomkins & Co
(86) International application number: PCT/US2018/019682
(87) International publication number: WO 2018/157044

(56) References cited:
- JP-A- S57 175 979
- US-A1- 2005 113 661
- US-A1- 2009 253 975
- US-A1- 2012 323 104
- US-A1- 2014 373 785

## Description

### Technical Field

The present invention generally relates to ECG devices.

### Background Art

The electrocardiogram (ECG) is an essential test that provides medical professionals with essential information in the management of patients with a variety of conditions. It is not only of significant importance in the evaluation and management of patients with chest pain, but also in patients with shortness of breath, syncope, dizziness, seizures, altered mental status, stroke, psychiatric conditions, overdose, palpitations and many other conditions. It is a bulky system with a multitude of wires and connections.

The ECG provides critical data to the health care provider in managing patients with multiple medical issues. The time to obtain this data is critical and often delayed by the current technology. Minutes can become critical in the patient with an acute myocardial infarction (heart attack).

Historically, there is training in the interpretation of ECG data, as well as placement of electrodes on the chest of each patient in anatomically specific positions.

Current ECG placement is done by technicians and providers of varying medical background, including paramedics, health care technicians, nursing assistants, nurses, and doctors. The current technology is bulky, with many wires and cables. The placement of the electrodes in the acquisition of an ECG is specific and requires special training. ECG acquisition is often limited and/or delayed by multiple factors such as body sweat, ability to transport the ECG device into confined areas, performance of concomitant medical procedures such as cardiopulmonary resuscitation (CPR). Because of many limitations, medical providers must make rapid decisions and potentially delay medical care while ECG testing is done. As emergency medicine providers, the inventors have identified a need for more rapid placement of the ECG electrodes, a more portable and manageable system that will not compromise medical care, and the need to eliminate electrode placement errors.

Sujdak, U.S. patent Number 6847836 for an Emergency ECG Electrode Chest Pad discloses a chest adapted for use in an emergency room.

Dominguez, U.S. Patent Number 6560473 for a Disposable ECG Chest Electrode Template With Built-in Defibrillation Electrodes discloses a template that carries ten electrodes.

US2005113661 discloses a precordial pad for positioning EKG electrodes on a patient for anatomically correct and repeatable placement. Data can be transmitted from the EKG pad of the invention by wire or wireless means. The pad includes a sizing aid and a positioning device.

Most of the prior art involves developing non-conforming devices that have to be sized independently and are impractical in the confined quarters of an ambulance. Most of the prior art does not address the ability to withstand the application to a chest wall that is diaphoretic or rapidly moving. The devices are bulky and often have a large footprint thereby obviating the application of other support devices or obscuring radiologic studies. There is very little attention to the ability to reduce the frequency of lead detachment. Nor is there much attention to conforming to multiple ECG recording devices which typically occurs during periods of transfer of care from pre-hospital to emergency department to inpatient units. The need to obtain serial measurements with a high degree of reproducibility is also missed by the prior art as subtle physiologic changes can suggest significant pathology warranting immediate intervention.

### Summary of the Invention

The present invention relates to an emergency cardiac and electrocardiogram (ECG) electrode placement device, as defined in the appended claims. For example, the emergency cardiac and electrocardiogram (ECG) electrode placement device

("EXG device") may incorporate electrical conducting materials and elastic material into a pad that is applied to the chest wall placing the electrodes in the appropriate anatomic locations on a patient. The EXG device solves the problem of lead detachment, lead reversal, inability to apply leads due to extremes in physiology, and lack of reproducibility to measure subtle changes. The ease of use with EXG device allows for acquisition of ECGs that would not have been obtained and therefore limits the opportunity loss of delays in diagnosis and treatment.

Creation of this device will reduce the time to complete an electrocardiogram (ECG) in the pre-hospital and emergency setting, eliminate systematic error in placement and interpretation of an ECG electrode, maintain and place electrodes in the proper anatomic locations across all body types, will not delay management in critical case, maintain proper skin contact through different physiologic responses such as sweat, cold and heat, as well as through medical treatment such as CPR, be easy to train providers in application and placement of ECG electrodes, and be adaptable to scenarios where space and situations limit ECG placement.

One aspect of the present invention is an emergency cardiac and electrocardiogram (ECG) electrode placement device comprising:
a body composed of a plurality of extension members, wherein the body comprises a main layer having a top surface, an adhesive layer, and a backing layer attached to an adhesive surface of the adhesive layer;
a plurality of electrodes, each of the plurality of electrodes positioned on the adhesive surface of the adhesive layer; and
an electrode connector cable extending from the body;
wherein each electrode of the plurality of electrodes is connected to the electrode connector cable through i) an electrical conducting elastic material incorporated into a top layer of the main layer, ii) through a plurality of printed wires wherein each printed wire of the plurality of printed wires is printed on the top surface of the main layer, or iii) through a plurality of cables with each cable of the plurality of cables positioned between the base layer and the adhesive layer.

In one embodiment, the device further comprises a cable management module comprising an upper cover, an upper guide piece, a lower guide piece and a lower cover wherein each of the upper guide piece and the lower guide piece has a plurality of channels therein, wherein each cable of the plurality of cables is routed through a channel of the plurality of channels. The plurality of extension members may comprise a first extension member, a second extension member, a third extension member, a fourth extension member and a fifth extension member, wherein each of the second extension member, the third extension member, the fourth extension member and the fifth extension member may extend outward from the cable management module. The first extension member may comprise a first electrode, a second electrode, a third electrode, a fourth electrode, a fifth electrode and a sixth electrode of the plurality of electrodes; wherein a seventh electrode of the plurality of electrodes may be positioned at a far end of the second extension member; wherein an eighth electrode of the plurality of electrodes may be positioned at a far end of the third extension member; wherein a ninth electrode of the plurality of electrodes may be positioned at a far end of the fourth extension member; and wherein a tenth electrode of the plurality of electrodes may be positioned at a far end of the fifth extension member.

### Brief Description of the Drawings

FIG. 1 is an illustration of an emergency cardiac and ECG electrode placement system used by a technician on a patient.
FIG. 2 is a top plan view of a first embodiment of an emergency cardiac and ECG electrode placement device in a storage state.
FIG. 3 is top plan view of a first embodiment of an emergency cardiac and ECG electrode placement device in an application state.
FIG. 4 is a side elevation view of a first embodiment of an emergency cardiac and ECG electrode placement device in a storage state.
FIG. 5 is a top plan view of a second embodiment of an emergency cardiac and ECG electrode placement device in an application state.
FIG. 6 is an isolated top plan view of a second embodiment of an emergency cardiac and ECG electrode placement device in an application state.
FIG. 7 is a top perspective view of a second embodiment of an emergency cardiac and ECG electrode placement device in an application state.
FIG. 8 is an isolated exploded view of a control module of a second embodiment of an emergency cardiac and ECG electrode placement device.
FIG. 9 is an isolated top perspective view of an electrode of an emergency cardiac and ECG electrode placement device.
FIG. 10 is an isolated exploded view of an electrode of an emergency cardiac and ECG electrode placement device.
FIG. 11 is an isolated cross-sectional view of an extension of is an isolated view of an electrode of an emergency cardiac and ECG electrode placement device.
FIG. 12 is a top perspective view of a connection module for an emergency cardiac and ECG electrode placement device.
FIG. 13 is an exploded top perspective view of a connection module for an emergency cardiac and ECG electrode placement device.
FIG. 14 is an isolated view of a side extension for an emergency cardiac and ECG electrode placement device.
FIG. 15A is an isolated view of a wireless emitter for an emergency cardiac and ECG electrode placement system.
FIG. 15B is an isolated view of a wireless receiver for an emergency cardiac and ECG electrode placement system.
FIG. 16 is a bottom plan view of a second embodiment of an emergency.
FIG. 17 is an isolated top plan view of a second embodiment of an emergency cardiac and ECG electrode placement device in an application state with an un-extended extension.
FIG. 17A is an isolated top plan view of a second embodiment of an emergency cardiac and ECG electrode placement device in an application state with an extended extension.
FIG. 18 is an illustration of a human body showing a chest skeleton and markers for electrode placement.
FIG. 19 is an illustration of a human body showing a chest skeleton and markers for electrode placement with an overlay of the emergency cardiac and ECG electrode placement device.
FIG. 20 is an illustration of a first embodiment of an emergency cardiac and ECG electrode placement device positioned on a patient.
FIG. 21 is an illustration of a second embodiment of an emergency cardiac and ECG electrode placement device positioned on a patient.
FIG. 22 is an illustration of a third embodiment of an emergency cardiac and ECG electrode placement device with a defibrillation mechanism positioned on a patient.
FIG. 22A is an illustration of a fourth embodiment of an emergency cardiac and ECG electrode placement device with a defibrillation mechanism positioned on a patient.
FIG. 22B is an illustration of a fifth embodiment of an emergency cardiac and ECG electrode placement device with a defibrillation mechanism positioned on a patient.
FIG. 22C is an illustration of a sixth embodiment of an emergency cardiac and ECG electrode placement device with a defibrillation mechanism positioned on a patient.
FIG. 23 is an illustration of a seventh embodiment of an emergency cardiac and ECG electrode placement device positioned on a patient.
FIG. 23A is an illustration of an eighth embodiment of an emergency cardiac and ECG electrode placement device positioned on a patient vice.
FIG. 23B is an illustration of a ninth embodiment of an emergency cardiac and ECG electrode placement device positioned on a patient.
FIG. 23C is an illustration of a tenth embodiment of an emergency cardiac and ECG electrode placement device positioned on a patient.
FIG. 24 is an illustration of an eleventh embodiment of an emergency cardiac and ECG electrode placement device positioned on a patient.
FIG. 24A is an illustration of a twelfth embodiment of an emergency cardiac and ECG electrode placement device positioned on a patient.
FIG. 25 is an isolated top perspective view of a top surface of an extension of an emergency cardiac and ECG electrode placement device.
FIG. 26 is an isolated bottom plan view of a bottom surface of an extension of an emergency cardiac and ECG electrode placement device.
FIG. 27 is an isolated top plan view of a top surface of an extension of an emergency cardiac and ECG electrode placement device.
FIG. 28 is an isolated top perspective view of a top surface of an extension of an emergency cardiac and ECG electrode placement device.
FIG. 28A is an isolated exploded cross-sectional view of the extension of an emergency cardiac and ECG electrode placement device of FIG. 28 and an electrode.
FIG. 28B is an isolated bottom view of an electrode for an emergency cardiac and ECG electrode placement device.
FIG. 28C is an isolated top view of an electrode for an emergency cardiac and ECG electrode placement device.
FIG. 29 is an isolated view of a portion of an emergency cardiac and ECG electrode placement device.
FIG. 30 is an isolated cross-sectional view of an bi-layer extension of with an electrode of a device.
FIG. 30A is an exploded view of FIG. 30.
FIG.31 is an isolated cross-sectional view of a single layer extension of with an electrode of a device.
FIG. 31A is an exploded view of FIG. 31.

### Best Mode(s) For Carrying Out The Invention

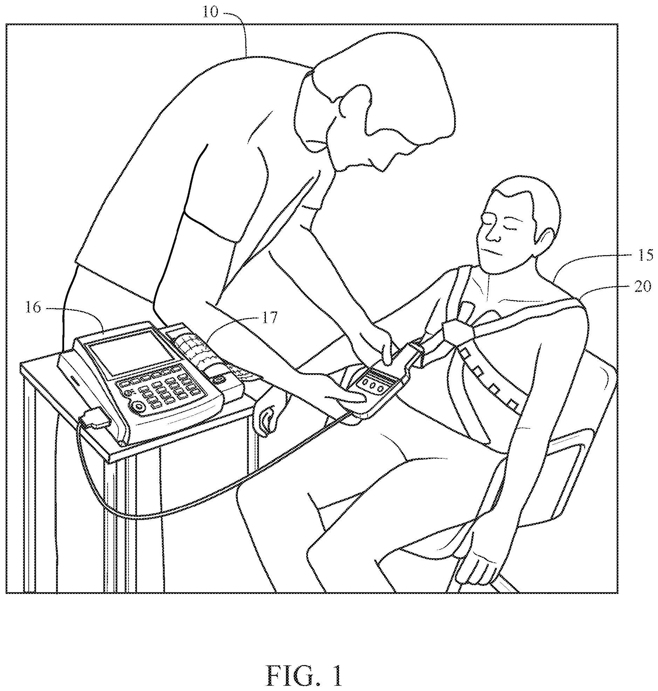

As shown in FIG. 1, the emergency cardiac and electrocardiogram (ECG) electrode placement device ("EXG device") 20 is a worn device that incorporates electrical conducting materials and elastic material into a pad that is applied to the chest wall placing the electrodes in the appropriate anatomic locations on a patient 15. A technician 10, such as an emergency responder, places the EXG device 20 on the patient 15 and connects the EXG device 20 to an ECG machine 16 which generates an ECG 17.

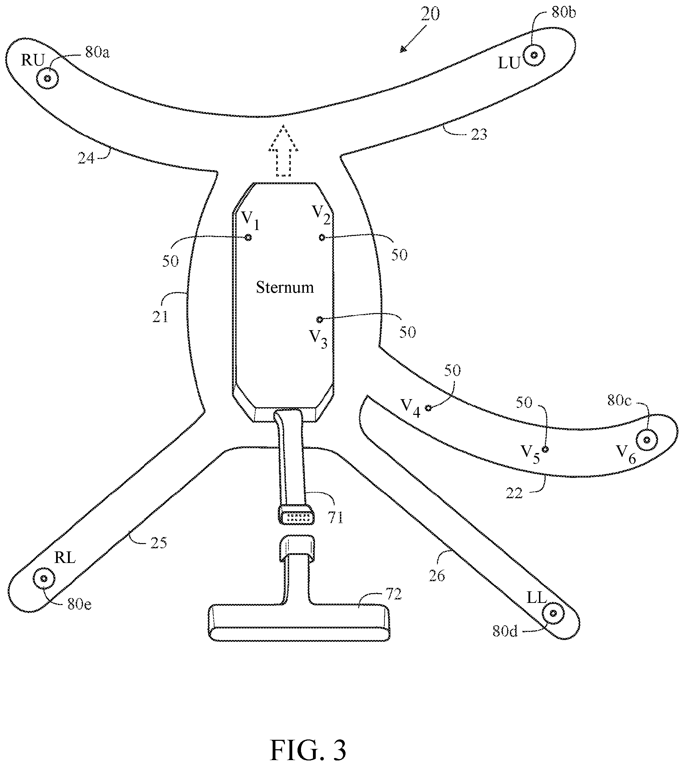

As shown in FIG. 3, the EXG device 20 preferably comprises a body 21, electrodes 50, cables 60 (not shown), and an electrode connector 71. The body 21 preferably comprises a first extension member 22, a second extension member 23, a third extension member 24, a fourth extension member 25 and a fifth extension member 26. The electrode connector 71 is positioned on the body 21. Each extension member 22-26 preferably has a width ranging from 1cm to 10cm, and a length ranging from 5cm to 20cm.

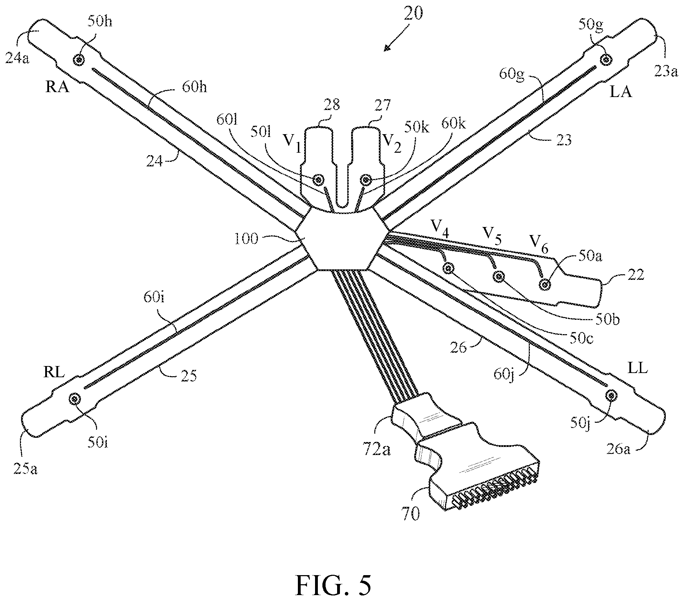

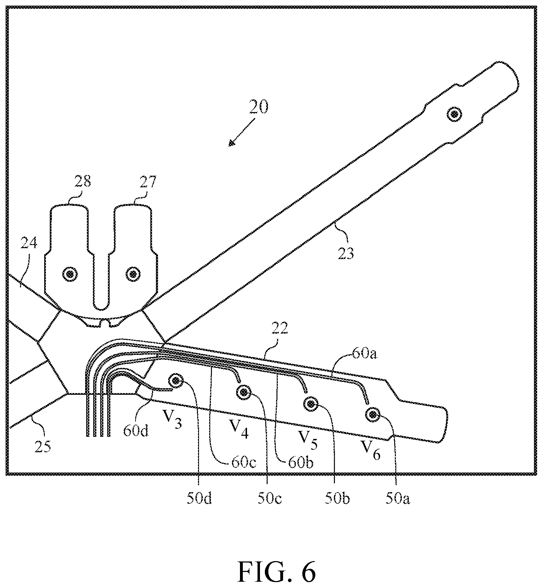

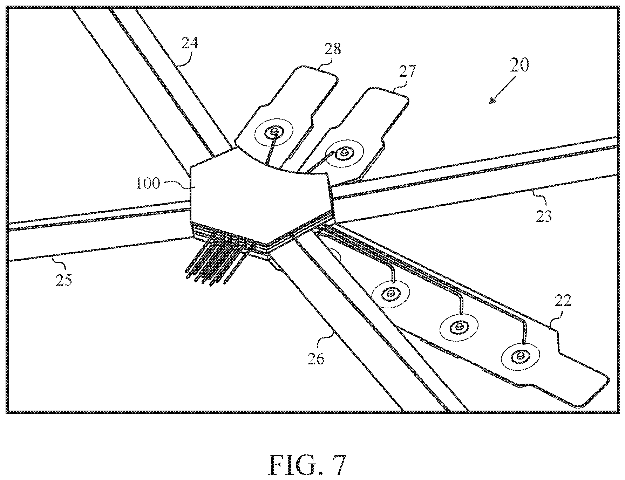

A second embodiment of EXG device 20 is shown in FIGS. 5-7. Each of the first extension member 22, the second extension member 23, the third extension member 24, the fourth extension member 25 and the fifth extension member 26 extends outward from the center of the body 21. The first extension member 22 preferably comprises a first electrode 50a, a second electrode 50b, a third electrode 50c, a fourth electrode 50d, a fifth electrode 50e (not shown) and a sixth electrode 50f (not shown). A cable 60a connects the electrode 50a to the electrode connector 71. A cable 60b connects the electrode 50b to the electrode connector 71. A cable 60c connects the electrode 50c to the electrode connector 71. A cable 60d connects the electrode 50d to the electrode connector 71. Although not shown, a cable 60e connects the electrode 50e to the electrode connector 71, and a cable 60f connects the electrode 50f to the electrode connector 71.

A seventh electrode 50g is positioned at a far end 23a of the second extension member 23, and a cable 60g connects the electrode 50g to the electrode connector 71. An eight electrode 50h is positioned at a far end 24a of the third extension member 24, and a cable 60h connects the electrode 50h to the electrode connector 71. A ninth electrode 50i is positioned at a far end 25a of the fourth extension member 25, and a cable 60i connects the electrode 50i to the electrode connector 71. A tenth electrode 50j is positioned at a far end 26a of the fifth extension member 26, and a cable 60j connects the electrode 50j to the electrode connector 71. The far ends 23a, 24a, 25a, 26a of the extension members 23, 24, 25, 26 and even the far end of extension member 22, act as strip extensions that assist in placing the electrode correctly. This strip extension is approximately 1 to 2 inches in length as measured from the electrode.

The EXG device 20 of FIG. 5 also comprises a sixth extension member 27 with an electrode 50k and a seventh extension member 28 with an electrode 50l. A cable 60k connects the electrode 50k to the electrode connector 71, and a cable 60i connects the electrode 50l to the electrode connector 71.

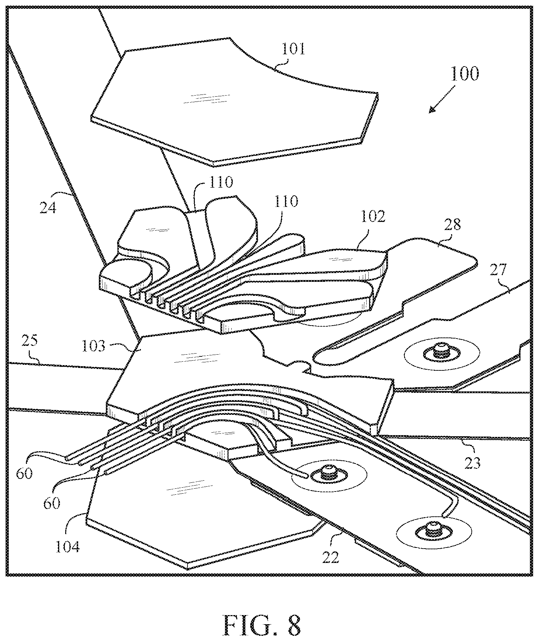

The EXG device 20 if FIGS. 5-8 includes a cable management module 100. The cable management module 100 comprises an upper cover 101, an upper guide piece 102, a lower guide piece 103 and a lower cover 104. Each of the upper guide piece 102 and the lower guide piece 103 has a plurality of channels 110 therein for guiding the cables 60 therethrough. The channels 110 of the cable management module 100 allow for the extension of an extension member to fit a patient, without the cables 60 becoming tangled.

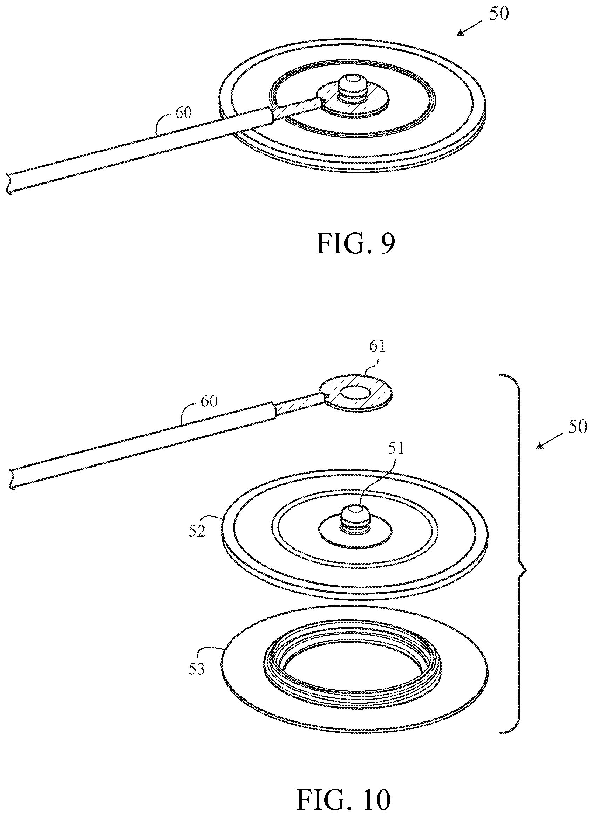

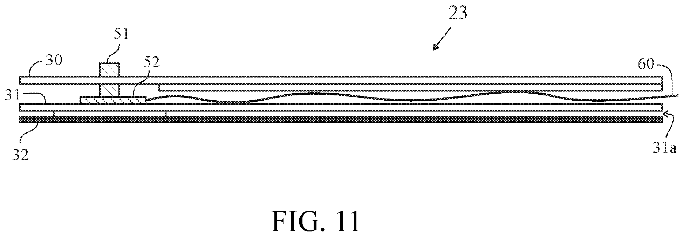

As shown in FIGS. 11, each extension member of the body 21 preferably comprises a base layer 30 composed of a flexible material, an adhesive layer 31 composed of a flexible material, and a backing layer 32 attached to an adhesive surface 31a of the adhesive layer 31. One preferred material for the flexible material is KT TAPE from Spidertech. The base layer 30 preferably has a Shore A hardness ranging from 50 to 90, which better allows for chest compressions. One preferred material for the adhesive layer is an adhesive from 3M. As shown in FIGS. 9-10, each of the electrodes 50 preferably comprises a connection stud 51, a contact pad interface 52 and a contact pad 53. Each contact pad 53 is preferably has a diameter ranging from 30milliimters ("mm") to 40mm, and most preferably 35mm, to in one embedment allow for retention of a gel protector. Each contact pad 53 is preferably composed of a material from 3M. A cable connector 61 of each cable 60 is connected to a connection stud 51 of each electrode 50 preferably using a conductive epoxy. Each cable connector 61 is preferably composed of 0.2mm thick copper, with a 26mm inside diameter. Each cable 60 of the plurality of cables 60 is positioned between the base layer 30 and the adhesive layer 31. Each cable 60 is connected to a corresponding electrode 50 of the plurality of electrodes 50 and connected to the electrode connector 71. Each cable 60 is preferably shielded to prevent electrical interference. Each of the plurality of cables preferably as an outer diameter ranging from 0.008 inch to 0.310 inch.

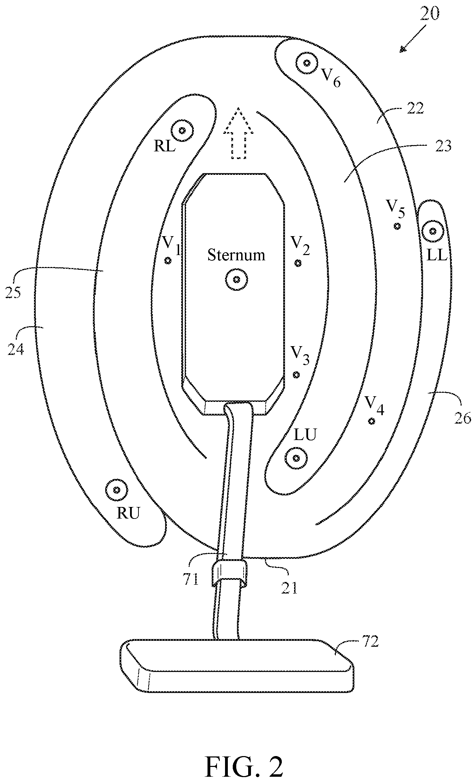

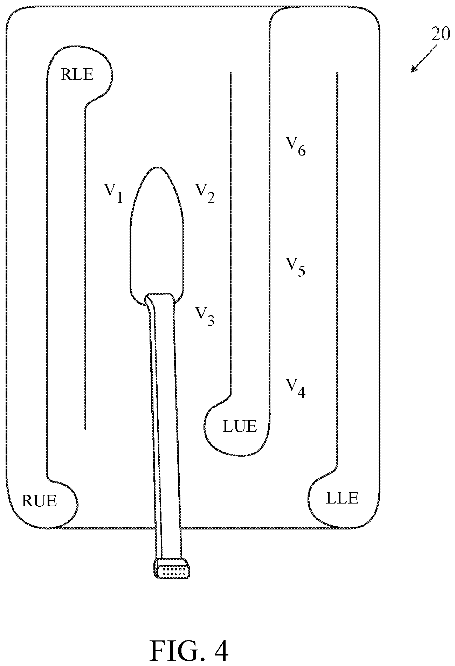

As shown in FIGS. 2 and 4, the EXG device 20 is preferably provided in a compact, easily stored and transported form, that is then applied to a patient's chest wall with materials that have adhesive capabilities that preferably resist moisture and conforms to the patient's body with inherent elasticity with placement of electrodes within a pad that maintain proper anatomic ratios and locations. The EXG device 20 preferably remains adherent to the patient's body through the duration of the acute pre-hospital and transition through the emergency department and acute hospitalization care periods (which is typically three days), but the EXG device 20 remains easily removable, while tolerating physiologic changes such as sweat, fever and medical treatment such as cardiac pulmonary resuscitation ("CPR"). The EXG device 20 is clearly marked and designed to fit to the chest wall so that its application ensures proper placement of all electrodes on the patient. The incorporated electrical conducting materials come together into a single cable/wire that is either directly or indirectly joined to an ECG monitoring device. The cable has adaptor capability that allows for wireless transfer of data to an ECG monitoring device obviating the need for having a bulky ECG machine in close proximity to the patient. The single cable also eliminates the need for multiple wires on a patient. Multiple wires that could potentially interfere with diagnostic imaging such as chest radiographs, or interfere with placement of emergency medical equipment such as transcutaneous cardiac pacer pads or defibrillating pad.

The EXG device 20 reduces the time to perform ECG testing significantly. With proper training, a user can anticipate ECG acquisition in less than one minute, and potentially within seconds. Current ECG data can take several minutes or longer depending on the care setting. It is not unusual for an ECG ordered in a hospital setting to take more than 10-30 minutes.

The EXG device 20 also eliminates lead transposition error. That is, the attachment of an electrode wire in a wrong electrode.

The EXG device 20 makes ECG data more reliable and reproducible. There is no variation in lead placement while performing serial ECGs --which is often done in the hospital and pre-hospital setting. The incorporated elastic electro-conductive materials allow for this small form factor to accommodate varying body types (man, women, adult, child, obese, anorexic) while maintaining strict anatomic ratios and correct placement and ensure proper lead placement.

The ease of use of the EXG device 20 makes ECG acquisition less inconvenient and potentially improves ECG utilization in the pre-hospital setting. The EXG device 20 also reduces the frequency of lead detachment. An alternative embedment of the EXG system has wireless transfer capability that makes acquisition of the ECG in any situation feasible. The EXG device 20 preferably incorporates either integrated elastic electro-conductive materials or printable elastic electro-conductive material used in the acquisition of electrical signals from the electrodes. The EXG device 20 adheres to skin surfaces through a variety of physiologic conditions not currently met by current methods. The EXG system allows for acquisition of data in settings that standard methods currently fail. Existing technology for ECG acquisition relies on technical expertise in lead placement. Removing technical error is dependent of operator knowledge and skill, as well as interpretation of ECG data to identify systemic error in placement.The time to acquire an ECG is dependent on many factors but is limited due to the number of electrodes that are placed on the chest and torso, which then need to be attached to the ECG device. There are preferably a minimum of ten wires involved, and more electrodes are possible to allow for more specific views of the right side of the heart and/or posterior heart leads.

The EXG device 20 is preferably a single device with embedded lead placement through a wearable material (such as a fabric) with a small physical footprint with the elasticity to maintain physiologic measurement across different ages, gender and body habitus without requiring multiple sized devices.

In one embodiment, the EXG device preferably comprises: adhesive stretchable material that is breathable and water/sweat resistant; embedded elastic electroconductive material conducting electrical signals from the integrated cardiac electrodes to a central data cable; embedded elastic electroconductive material/wiring/cabling arranged to allow for stretching across body types and sizes; electrode connection port; Bluetooth capable emitter and receiver; conduction gel; and embedded electrodes (manufactured or printable).

The elastic adhesive membrane preferably provides adherence to body surface. It is preferably tolerant to moisture. The EXG device preferably incorporates electroconductive materials and electrodes that conduct signal from the skin to a single data cable/wire. The EXG device preferably expands in an elastic fashion to appropriately fit varied body types while meeting exact ratios of electrode distance without distortion. The EXG device preferably has significant stability of size and shape with elastic components to make it easily applicable to the chest wall. The EXG device preferably comes in a compact form factor.

In one embodiment, there is encapsulated expandable electroconductive material within the membrane. Within the elastic membrane is incorporated electroconductive materials that originate from each electrode to come together into a single data cable encompassing a minimum often ECG wires to allow for a standard twelve lead ECG (by convention there are two leads that are inferred from the ten connections).

Alternatively, the EXG device allows for the use of external electrodes. In the event that ECG monitoring equipment is not compatible with the data cable, electrodes at the ascribed anatomical locations can be accessed with standard medical cardiac monitoring and ECG devices.

In one embodiment, there is a conductive membrane at ECG electrode sites. At the ascribed electrode ECG locations is a typical electroconductive Ag/AgCL membrane to conduct current from body surface to ECG monitoring device.

In one embodiment, a data cable brings individual electrodes into one cable that encompasses a minimum often wires/leads of the typical ECG analysis which is then compatible with various ECG devices and wireless transfer system. Other conductive interfaces may be utilized with the invention including ones composed of graphene/carbon, nickel, and copper.

In use, one applies the EXG device 20 to an anterior chest wall overlying the sternum symmetrically at a level above the nipple line of the patient and below the sternal notch, removing the backing layer 32 to expose the adhesive surface 31a of the adhesive layer 31. The precordial limb is then stretched to the lateral chest wall at the mid axillary line below the nipple line. Similarly each limb will have the backing layer 32 removed in succession to expose the adhesive surface 31a of the adhesive layer 31. The right upper extremity limb is stretched towards the right shoulder. The left upper extremity is stretched towards the left shoulder. The right lower extremity limb is stretched to the right lower abdominal quadrant. The left lower extremity limb is stretched to the left lower abdominal quadrant. The cable is either attached to directly to the ECG device cable. Or in versions utilizing a BLUETOOTH transceiver, then the EXG device 20 is activated to sync with the BLUETOOTH transceiver that is already connected to the ECG device.

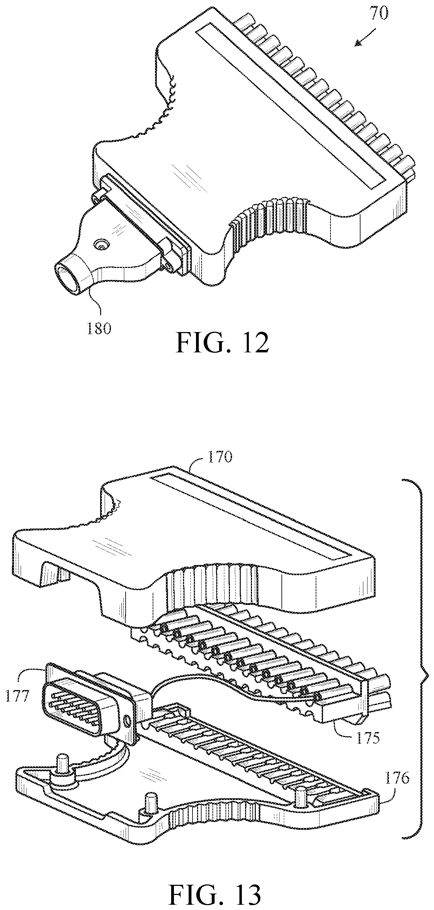

A preferred embodiment of a connector module 70 is shown in FIGS. 12-13. The connector module 70 preferably comprises a top cover portion 170, a bottom cover portion 176, a plurality of connector pins 175 and a fifteen pins sub connector 177. An interface connector 180 is also shown.

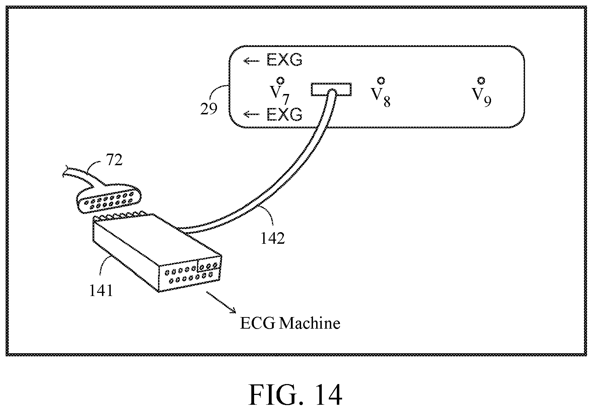

A posterior extension member 29 is shown in FIG. 14. This additional posterior extension member 29 preferably has multiple electrodes that connect via cable 142 to an intermediary adapter module 141 which connects to the electrode connector 72. The posterior leads preferably are connected through the adapter module 141 onto the end of the original EXG device 20 and basically take over leads V5-6 for the standard ECG.

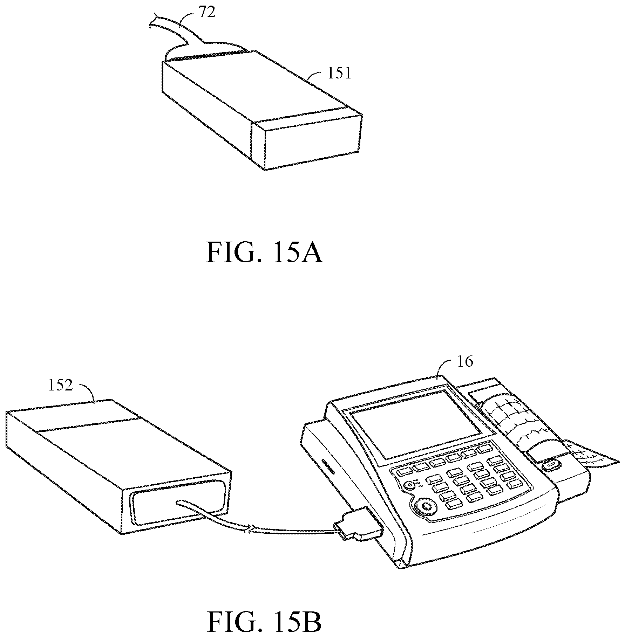

As shown in FIGS. 15A and 15B, in an alternative embodiment, the EXG device 20 comprises a wireless emitter 151 and a wireless receiver 152. The wireless emitter 151 is connected to electrode connector 72, and the wireless receiver is connected to the ECG machine 16. The wireless emitter 151 and the wireless receiver 152 preferably operation on a BLUETOOTH communication protocol.

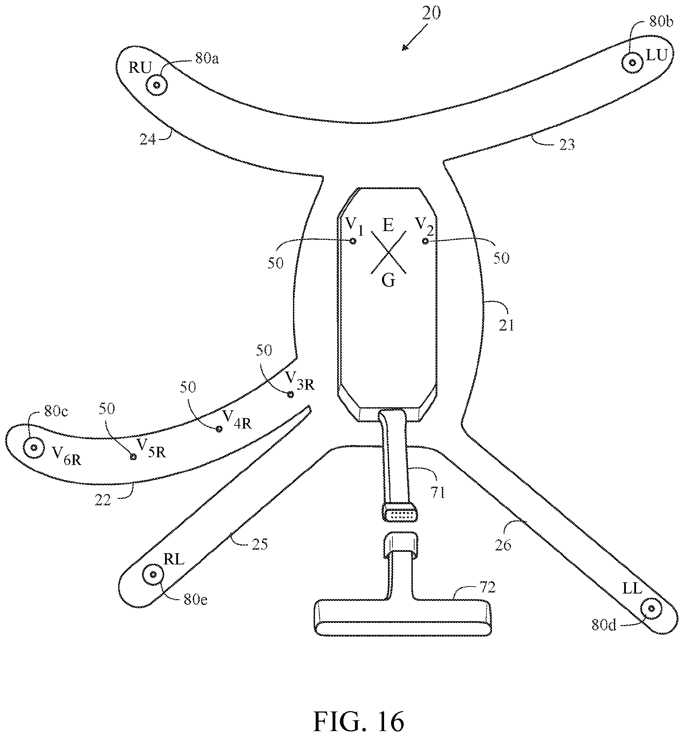

As shown in FIG. 16, the EXG device 20 also preferably comprises a plurality of external electrodes 80. A third extension member 24 comprises a first external electrode 80a. A second extension member 23 comprises a second external electrode 80b. A first extension member 22 comprises a third external electrode 80c. A fifth extension member 26 comprises a fourth external electrode 80d. A fourth extension member 25 comprises a fifth external electrode 80e.

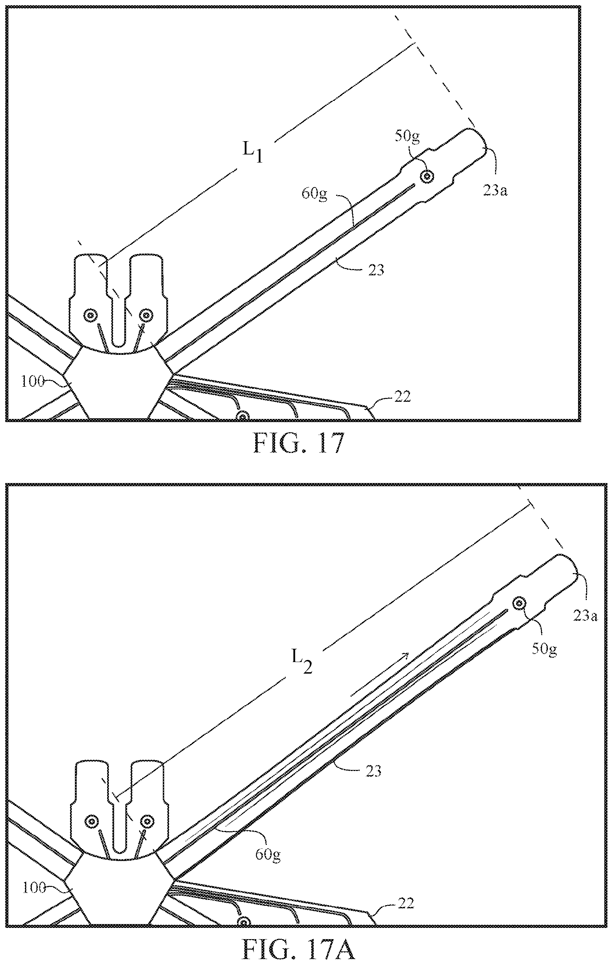

FIGS. 17 and 17A illustrate the stretching capability of the extension members of the EXG device 20. The extension member 23 extends from a length L1 (as shown in FIG. 17) to a length L2 (as shown in FIG. 17A). In a preferred embodiment, each extension member 23, 24, 25 and 26 extends from a length L1 ranging from 7.0 to 14.0 inches to a length L2 ranging from 10.0 to 16.5 inches. In a most preferred embodiment, L1 ranges from 10 to 11 inches, and L2 ranges from 12 to 13 inches. A width of each extension member 22, 23, 24, 25, 26 preferably ranges from 1centimeter ("cm") to 10cm, and most preferably 2.5cm to 5cm. A thickness of each extension member 22, 23, 24, 25, 26 preferably ranges from 0.1 inch to 0.5 inch.

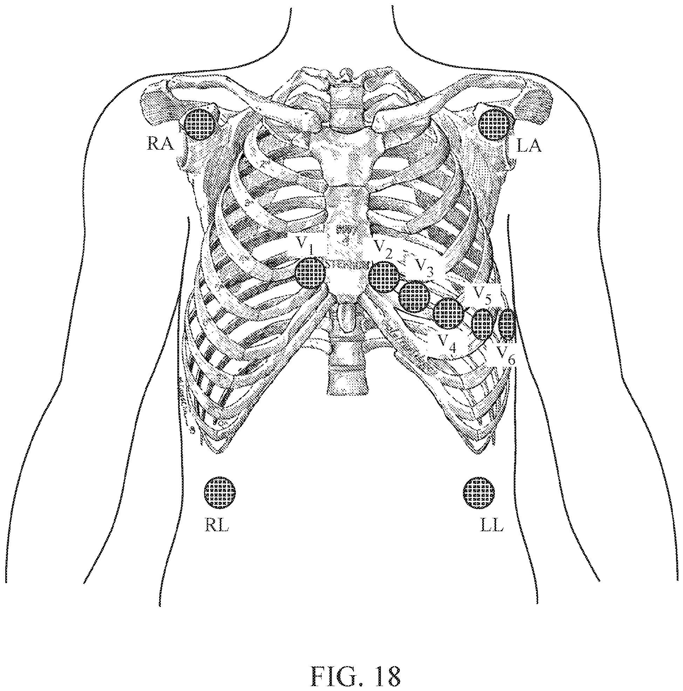

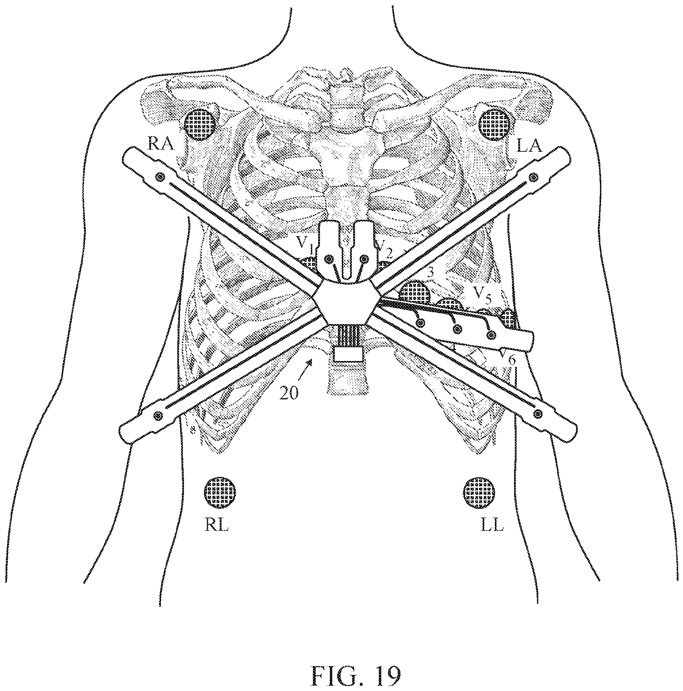

FIG. 18 is an illustration of a human body showing a chest skeleton and markers for electrode placement. FIG. 19 is an illustration of a human body showing a chest skeleton and markers for electrode placement with an overlay of the emergency cardiac and ECG electrode placement device 20.

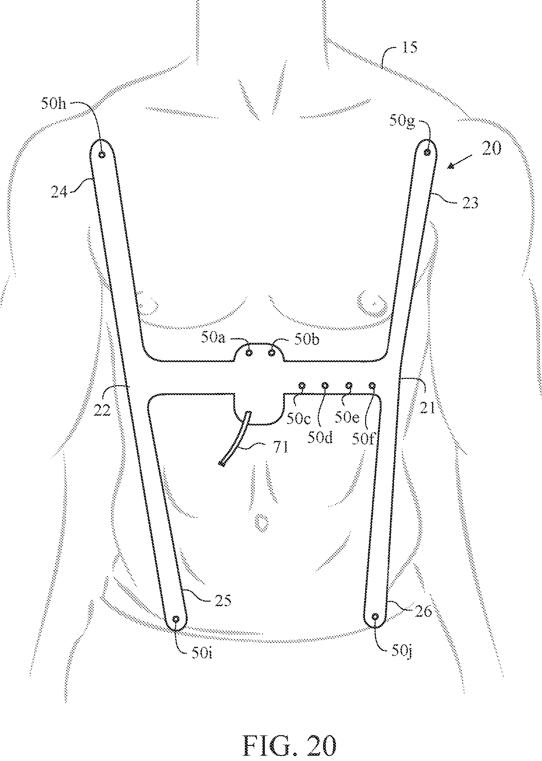

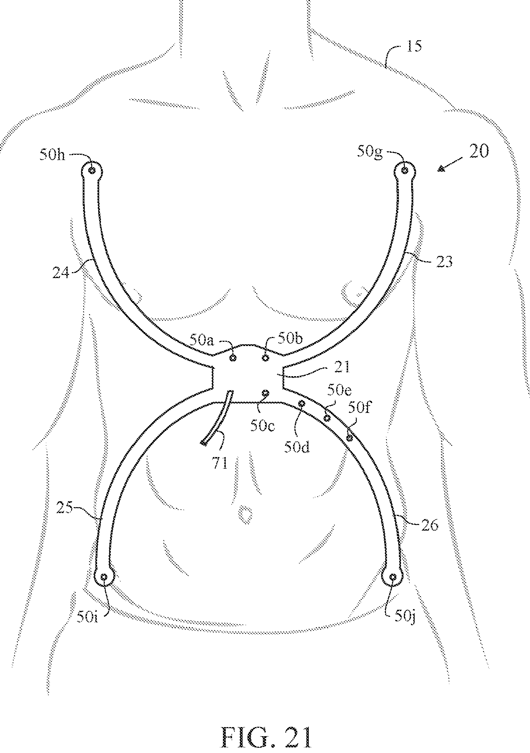

As shown in FIGS. 20 and 21, the EXG device 20 preferably comprises a body 21, electrodes 50, printed wires or an electrical conducting flexible material 60 (not shown), and an electrode cable connector 71. The body 21 preferably comprises a center extension member 22, a first extension member 23, a second extension member 24, a third extension member 25 and a fourth extension member 26. The electrode cable connector 71 is positioned on the body 21. Each extension member 22-26 preferably has a width ranging from 1cm to 10cm, and a length ranging from 5cm to 20cm. The center extension member 22 preferably comprises a first electrode 50a, a second electrode 50b, a third electrode 50c, a fourth electrode 50d, a fifth electrode 50e and a sixth electrode 50f. Printed wires or electrical conducting flexible material 60 (not shown) connect each electrode 50 to the electrode cable connector 71.

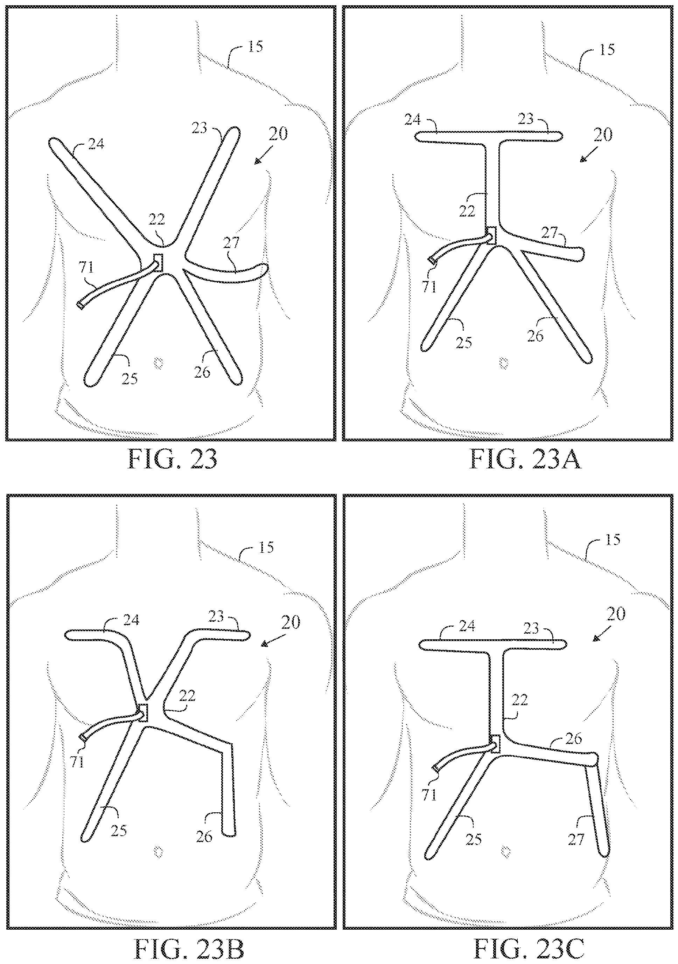

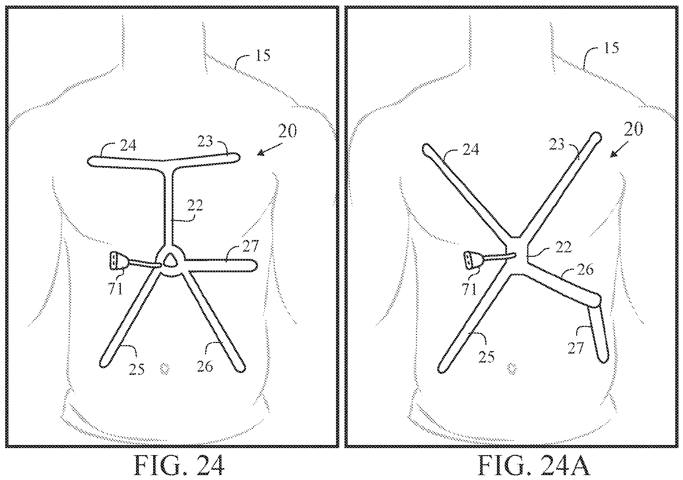

Other embodiments of EXG device 20 are shown in FIGS. 23, 23A, 23B, 23C, 24 and 24A. The extension members extend outward from the center of the body 21.

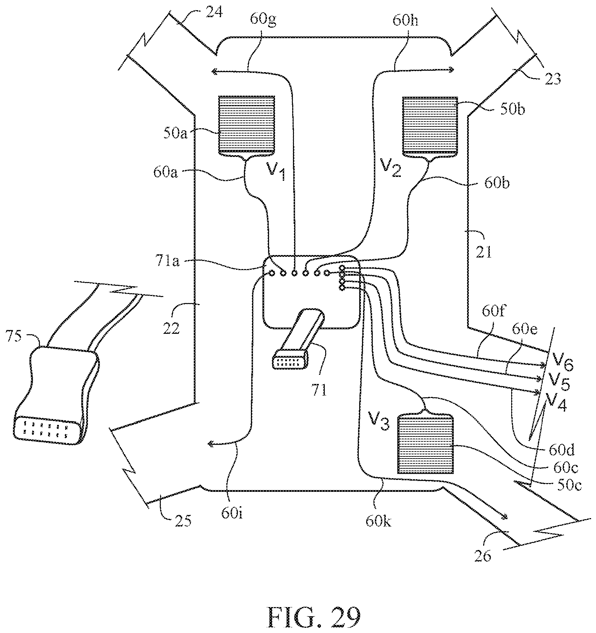

As shown in FIG. 29, a printed wire 60a connects the electrode 50a to the electrode cable connector 71. A printed wire 60b connects the electrode 50b to the electrode cable connector 71. A printed wire 60c connects the electrode 50c to the electrode cable connector 71. A printed wire 60d connects the electrode 50d to the electrode cable connector 71. A printed wire 60e connects the electrode 50e to the electrode cable connector 71. A printed wire 60f connects the electrode 50f to the electrode cable connector 71. A printed wire 60g connects the electrode 50g to the electrode cable connector 71. A printed wire 60h connects the electrode 50h to the electrode cable connector 71. A printed wire 60i connects the electrode 50i to the electrode cable connector 71. A printed wire 60j connects the electrode 50j to the electrode cable connector 71. A ten pin electrode interface 75 connects to the electrode cable connector 71. On one embodiment, the elastic electrically conductive material is preferably applied with a 3D printer directly on the main layer.

Alternatively, an elastic conductive material is substituted for each of the printed wires in FIG. 29. Such elastic conductive materials preferably comprise silver chloride and/or graphene. The body 21 is preferably composed of a kinesiology type tape.

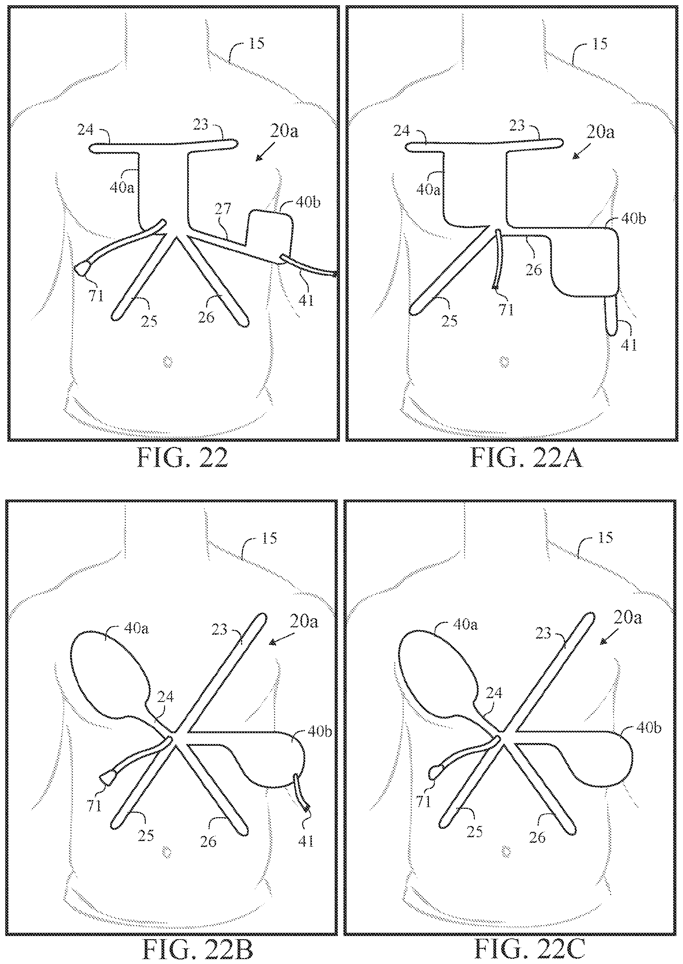

Alternative embodiments of the EXG device 20a shown in FIGS. 22, 22A, 22B, and 22C also comprise integrated defibrillation pads 40a and 40b connected to a defibrillation cable 41. In the unstable patient, defibrillation becomes a crucial aspect of emergency cardiac care. The use of defibrillation pads has in the field historically been done with pad placement at the discretion of the first responder/paramedic. The most common deployment being anteriorly. This often leads to suboptimal placement and suboptimal delivery of electricity. The EXG-DF with defibrillator pad assures proper placement of the device in the anterior posterior configuration, which allows for optimal electrical conductance to the heart. The vector of electrical conductance is optimally placed in an anterior posterior configuration. There is no device that provides optimal defibrillator pad placement while integrating twelve lead EKG ability with ability to extend to include posterior and right sided lead EKG. The ability to obtain instant EKG data after critical defibrillation has heretofore been impractical for the pre-hospital care provider. The EXG-DF-DF addresses this critical issue in cardiac care.

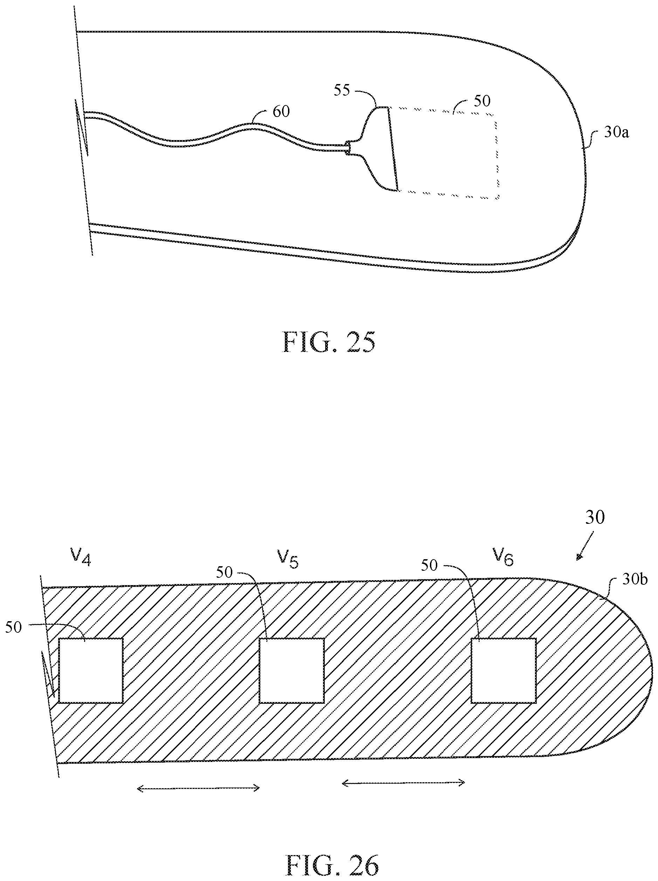

FIG. 25 illustrates an isolated top perspective view of a top surface of an extension of the EXG device 20. The extension has a top layer 30a with an integrated printed wire (or elastic electrical conducting material) 60 connected to an electrode interface 55 integrated with an electrode 50 that is positioned on an adhesive surface below. The electrode 50 is not positioned on the top surface 30a of the main layer 30.

FIG. 26 illustrates an isolated bottom plan view of a bottom surface of an extension of an EXG device 20. On bottom adhesive surface 30b of the main layer 30 has electrodes 50 positioned thereon.

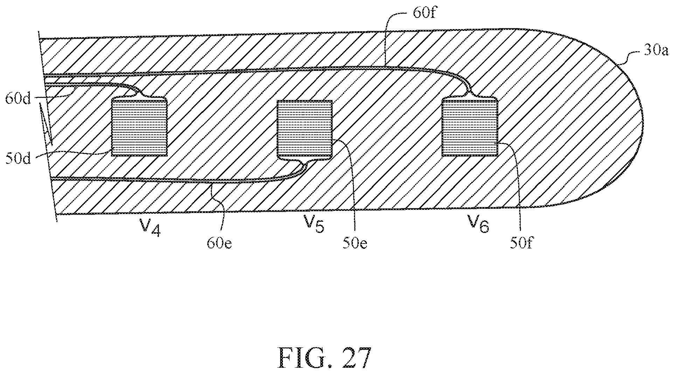

FIG. 27 illustrates an isolated top plan view of a top surface of an extension of the EXG device 20. The main layer 30 of the extension has a top layer 30a with an integrated printed wires (or elastic electrical conducting material) 60d, 60e and 60f connected to corresponding electrodes 50d, 50e and 50f that are positioned on an adhesive surface below. The electrodes 50d, 50e and 50f are not positioned on the top surface 30a of the main layer 30.

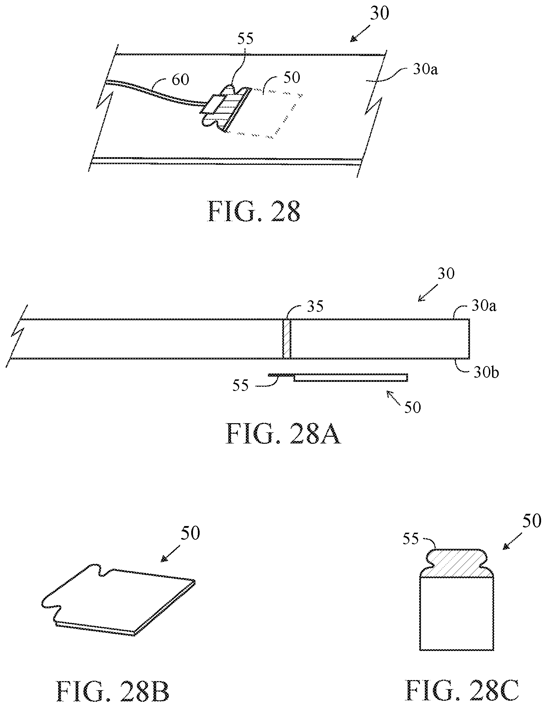

FIG. 28 is an isolated top perspective view of a top surface of an extension of the EXG device 20. The extension has a top layer 30a with an integrated printed wire (or elastic electrical conducting material) 60 connected to an electrode interface 55 integrated with an electrode 50 that is positioned on an adhesive surface below. The electrode 50 is not positioned on the top surface 30a of the main layer 30. FIG. 28A is an isolated exploded cross-sectional view of the extension of the EXG device 20 of FIG. 28 and an electrode 50. The interface 55 is placed through an aperture 35 in the main layer 30 to connect to the integrated printed wire (or elastic electrical conducting material) 60. FIG. 28B is an isolated bottom view of an electrode 50 for an EXG device 20. FIG. 28C is an isolated top view of an electrode 50 with an interface 55 for an EXG device 20. The interface is preferably composed of a conductive material such as graphene or silver chloride. The electrode 50 is preferably composed of a silver chloride material.

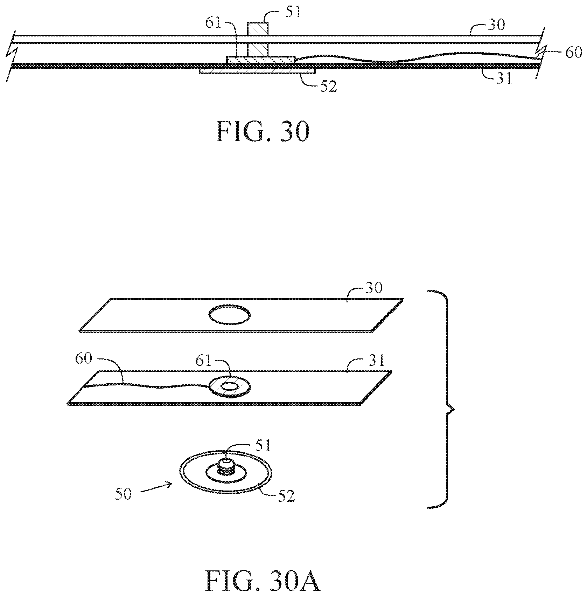

A bi-layer extension is shown in FIGS. 30 and 30A. Each extension member of the body 21 preferably comprises a top layer 30 composed of a flexible material and an adhesive layer 31 composed of a flexible material, with a removable backing layer attached to an adhesive surface of the adhesive layer 31. A top surface of the adhesive layer preferably includes an integrated printed wire (or elastic electrical conducting material) 60 with a connector 61. One preferred material for the flexible material is KT TAPE from Spidertech. The top layer 30 preferably has a Shore A hardness ranging from 50 to 90, which better allows for chest compressions. One preferred material for the adhesive layer is an adhesive from 3M. Each of the electrodes 50 preferably comprises a connection stud 51and a contact pad 52. Each contact pad 52 is preferably has a diameter ranging from 30milliimters ("mm") to 40mm, and most preferably 35mm, to in one embedment allow for retention of a gel protector. Each contact pad 52 is preferably composed of a material from 3M. A cable connector 61 is connected to a connection stud 51 of each electrode 50 preferably using a conductive epoxy. Each cable connector 61 is preferably composed of 0.2mm thick copper, with a 26mm inside diameter.

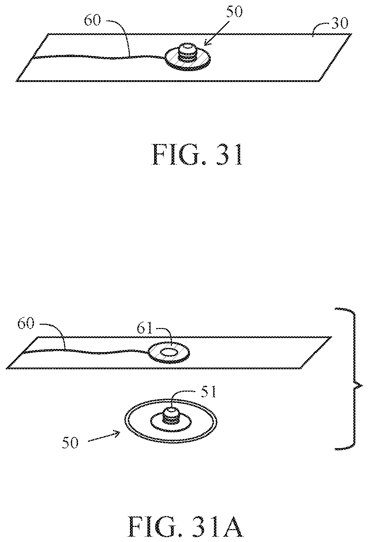

FIGS. 31 and 31A illustrate an isolated cross-sectional view of a single layer extension. A top surface of the main layer 30 has an integrated printed wire (or elastic electrical conducting material) 60 with a connector 61. Each electrode 50 is attached to an adhesive surface of the main layer 30 with a stud extending through an aperture to connect to the connector 61.

A preferred source for the printed wires is PE874 conductor ink from Intexar Dupont. A conductive elastic rubber material is disclosed in U.S. Patent Number 8491884. A stretchable graphene film material is disclosed in Chen et al., U.S. Patent Publication Number 20150273737. A flexible conductive material comprising silver is disclosed in Taguchi et al., U.S. Patent Publication Number 20130056249.

The emergency cardiac and ECG electrode placement device 20 is capable of being applied to a patient while an emergency vehicle is in motion since the device 20 is applied to and adheres to a patient's chest area, which mitigates signal loss. Likewise, the emergency cardiac and ECG electrode placement device 20 is capable of being applied to a patient that is moving due to a seizure, aggressiveness, and the like.

## Claims

1. An emergency cardiac and electrocardiogram, ECG, electrode placement device, the device comprising:
a body composed of a plurality of extension members, wherein the body comprises a main layer having a top surface, an adhesive layer, and a backing layer attached to an adhesive surface of the adhesive layer;
a plurality of electrodes, each of the plurality of electrodes positioned on the adhesive surface of the adhesive layer; and
an electrode connector cable extending from the body;
wherein each electrode of the plurality of electrodes is connected to the electrode connector cable through i) an electrical conducting elastic material incorporated into a top layer of the main layer, ii) through a plurality of printed wires wherein each printed wire of the plurality of printed wires is printed on the top surface of the main layer, or iii) through a plurality of cables with each cable of the plurality of cables positioned between the base layer and the adhesive layer.

2. The device according to claim 1 wherein each extension member of the plurality of extension members has a width ranging from 1cm to 10cm, and a length ranging from 5cm to 20cm.

3. The device according to claim 1 wherein each of the plurality of electrodes is composed of an AgCl gel electrode plate.

4. The device according to claim 1 wherein the plurality of extension members comprises a center extension member, first extension member, a second extension member, a third extension member, and a fourth extension member.

5. The device according to claim 1 wherein the printed wire is composed of a conducting ink or printed electrically conductive material.

6. The device according to claim 1 wherein the electrical conducting elastic material is graphene yarn, silver/silver-chloride wire, or a conductive rubber.

7. The device according to claim 1 further comprising a cable management module comprising an upper cover, an upper guide piece, a lower guide piece and a lower cover wherein each of the upper guide piece and the lower guide piece has a plurality of channels therein, wherein each cable of the plurality of cables is routed through a channel of the plurality of channels.

8. The device according to claim 7 wherein the plurality of extension members comprises a first extension member, a second extension member, a third extension member, a fourth extension member and a fifth extension member, wherein each of the second extension member, the third extension member, the fourth extension member and the fifth extension member extends outward from the cable management module.

9. The device according to claim 8 wherein the first extension member comprises a first electrode, a second electrode, a third electrode, a fourth electrode, a fifth electrode and a sixth electrode of the plurality of electrodes; wherein a seventh electrode of the plurality of electrodes is positioned at a far end of the second extension member; wherein an eighth electrode of the plurality of electrodes is positioned at a far end of the third extension member; wherein a ninth electrode of the plurality of electrodes is positioned at a far end of the fourth extension member; and wherein a tenth electrode of the plurality of electrodes is positioned at a far end of the fifth extension member.

10. The device according to claim 9 wherein each extension member has a width ranging from 1cm to 10cm, and a length ranging from 5cm to 20cm.

11. The device according to claim 8 further comprising a plurality of external electrodes.

12. The device according to claim 8 further comprising a sixth extension member and a seventh extension member, each of the sixth extension member and a seventh extension member having an electrode positioned thereon.

## Patentansprüche

1. Ein Notfall-Herz- und Elektrokardiogramm, EKG, Elektrodenplatzierung Vorrichtung, wobei die Vorrichtung umfasst:
einen Körper, der aus einer Vielzahl von Verlängerungselementen zusammengesetzt ist,
wobei der Körper eine Hauptschicht mit einer Oberseite, eine Klebeschicht und eine an einer Klebefläche der Klebeschicht angebrachte Rückseitenschicht umfasst;
eine Vielzahl von Elektroden, wobei jede der Vielzahl von Elektroden auf der Klebefläche der Klebeschicht angeordnet ist; und
ein Elektrodenanschlusskabel, das vom Körper ausgeht;
wobei jede Elektrode der Vielzahl von Elektroden mit dem Elektrodenanschlusskabel verbunden ist durch i) ein elektrisch leitendes elastisches Material, das in eine obere Schicht der Hauptschicht eingearbeitet ist, ii) durch eine Vielzahl von gedruckten Drähten, wobei jeder gedruckte Draht der Vielzahl von gedruckten Drähten auf die obere Oberfläche der Hauptschicht gedruckt ist, oder iii) durch eine Vielzahl von Kabeln, wobei jedes Kabel der Vielzahl von Kabeln zwischen der Basisschicht und der Klebeschicht angeordnet ist.

2. Vorrichtung nach Anspruch 1, bei der jedes Verlängerungselement der Vielzahl von Verlängerungselementen eine Breite im Bereich von 1 cm bis 10 cm und eine Länge im Bereich von 5 cm bis 20 cm aufweist.

3. Vorrichtung nach Anspruch 1, wobei jede der mehreren Elektroden aus einer AgCl-Gelelektrodenplatte besteht.

4. Vorrichtung nach Anspruch 1, bei der die Mehrzahl der Verlängerungselemente ein mittleres Verlängerungselement, ein erstes Verlängerungselement, ein zweites Verlängerungselement, ein drittes Verlängerungselement und ein viertes Verlängerungselement umfasst.

5. Vorrichtung nach Anspruch 1, wobei der gedruckte Draht aus einer leitenden Tinte oder einem gedruckten elektrisch leitenden Material besteht.

6. Vorrichtung nach Anspruch 1, wobei das elektrisch leitende elastische Material ein Graphengarn, ein Silber/Silberchlorid-Draht oder ein leitfähiger Gummi ist.

7. Die Vorrichtung nach Anspruch 1 umfasst ferner ein Kabelmanagementmodul, das eine obere Abdeckung, ein oberes Führungsteil, ein unteres Führungsteil und eine untere Abdeckung umfasst, wobei sowohl das obere Führungsteil als auch das untere Führungsteil eine Vielzahl von Kanälen aufweist, wobei jedes Kabel der Vielzahl von Kabeln durch einen Kanal der Vielzahl von Kanälen geführt wird.

8. Vorrichtung nach Anspruch 7, wobei die mehreren Verlängerungselemente ein erstes Verlängerungselement, ein zweites Verlängerungselement, ein drittes Verlängerungselement, ein viertes Verlängerungselement und ein fünftes Verlängerungselement umfassen, wobei sich das zweite Verlängerungselement, das dritte Verlängerungselement, das vierte Verlängerungselement und das fünfte Verlängerungselement jeweils von dem Kabelmanagementmodul nach außen erstrecken.

9. Vorrichtung nach Anspruch 8, wobei das erste Verlängerungselement eine erste Elektrode, eine zweite Elektrode, eine dritte Elektrode, eine vierte Elektrode, eine fünfte Elektrode und eine sechste Elektrode der Vielzahl von Elektroden umfasst; wobei eine siebte Elektrode der Vielzahl von Elektroden an einem entfernten Ende des zweiten Verlängerungselements angeordnet ist; wobei eine achte Elektrode der Vielzahl von Elektroden an einem entfernten Ende des dritten Verlängerungselements angeordnet ist; wobei eine neunte Elektrode der Vielzahl von Elektroden an einem entfernten Ende des vierten Verlängerungselements angeordnet ist; und
wobei eine zehnte Elektrode aus der Vielzahl der Elektroden an einem entfernten Ende des fünften Verlängerungselements angeordnet ist.

10. Vorrichtung nach Anspruch 9, wobei jedes Verlängerungselement eine Breite im Bereich von 1 cm bis 10 cm und eine Länge im Bereich von 5 cm bis 20 cm aufweist.

11. Vorrichtung nach Anspruch 8 umfasst ferner eine Vielzahl von externen Elektroden.

12. Vorrichtung nach Anspruch 8 umfasst ferner ein sechstes Verlängerungselement und ein siebtes Verlängerungselement, wobei sowohl das sechste Verlängerungselement als auch das siebte Verlängerungselement mit einer darauf befindlichen Elektrode versehen ist.

## Revendications

1. Un dispositif de mise en place d'urgence d'électrodes cardiaques et d'électrocardiogramme, ECG, le dispositif comprenant :
un corps constitué d'une pluralité d'éléments d'extension, le corps comprenant une couche principale ayant une surface supérieure, une couche adhésive et une couche de support attachée à une surface adhésive de la couche adhésive ;
une pluralité d'électrodes, chacune de la pluralité d'électrodes étant positionnée sur la surface adhésive de la couche adhésive ; et
un câble de connexion d'électrodes s'étendant à partir du corps;
chaque électrode de la pluralité d'électrodes étant reliée au câble de connexion d'électrodes par i) un matériau conducteur d'électricité élastique incorporé dans une couche supérieure de la couche principale, ii) par une pluralité de fils imprimés, chaque fil imprimé de la pluralité de fils imprimés étant imprimé sur la surface supérieure de la couche principale, ou iii) par une pluralité de câbles, chaque câble de la pluralité de câbles étant positionné entre la couche de base et la couche adhésive.

2. Dispositif selon la revendication 1, chaque élément d'extension de la pluralité d'éléments d'extension ayant une largeur allant de 1 cm à 10 cm et une longueur allant de 5 cm à 20 cm.

3. Dispositif selon la revendication 1, chacune de la pluralité d'électrodes étant constituée d'une plaque d'électrode en gel AgCl.

4. Dispositif selon la revendication 1, la pluralité d'éléments d'extension comprenant un élément d'extension central, un premier élément d'extension, un deuxième élément d'extension, un troisième élément d'extension et un quatrième élément d'extension.

5. Dispositif selon la revendication 1, le fil imprimé étant constitué d'une encre conductrice ou d'un matériau imprimé conducteur d'électricité.

6. Dispositif selon la revendication 1, le matériau conducteur d'électricité élastique étant du fil de graphène, un fil argent/chlorure d'argent ou un caoutchouc conducteur.

7. Dispositif selon réclamation 1, comprenant en outre un module de gestion de câbles comportant un cache supérieur, une pièce de guidage supérieure, une pièce de guidage inférieure et un cache inférieur, chacune des pièces de guidage supérieure et pièce de guidage inférieure ayant une pluralité de canaux en son intérieur, chaque câble de la pluralité de câbles étant passé par un canal de la pluralité de canaux.

8. Dispositif selon la revendication 7, la pluralité d'éléments d'extension comprenant un premier élément d'extension, un deuxième élément d'extension, un troisième élément d'extension, un quatrième élément d'extension et un cinquième élément d'extension, chacun des deuxième élément d'extension, troisième élément d'extension, quatrième élément d'extension et cinquième élément d'extension s'étendant du module de gestion de câbles vers l'extérieur.

9. Dispositif selon la revendication 8, le premier élément d'extension comprenant une première électrode, une deuxième électrode, une troisième électrode, une quatrième électrode, une cinquième électrode et une sixième électrode de la pluralité d'électrodes, une septième électrode de la pluralité d'électrodes étant positionnée à une extrémité éloignée du deuxième élément d'extension ; une huitième électrode de la pluralité d'électrodes étant positionnée à une extrémité éloignée du troisième élément d'extension ; une neuvième électrode de la pluralité d'électrodes étant positionnée à une extrémité éloignée du quatrième élément d'extension ; et une dixième électrode de la pluralité d'électrodes état positionnée à une extrémité éloignée du cinquième élément d'extension.

10. Dispositif selon la revendication 9, chaque élément d'extension ayant une largeur allant de l cm à 10 cm et une longueur allant de 5 cm à 20 cm.

11. Dispositif selon la revendication 8, comprenant en outre une pluralité d'électrodes externes.

12. Dispositif selon la revendication 8, comprenant en outre un sixième élément d'extension et un septième élément d'extension, chacun des sixième élément d'extension et septième élément d'extension ayant une électrode positionnée là-dessus.
